# EUROPEAN PATENT APPLICATION

(11) **EP 4 361 164 A1**
(43) Date of publication of application: **01.05.2024**
(21) Application number: 22815278.1
(22) Date of filing: 31.05.2022
(51) Int. Cl.: C07H 13/04, C07H 1/06, A61K 31/7024, A61P 19/00, A61P 19/02, A61P 19/10, A61P 29/00

(54) **CRYSTAL FORMS OF GLUCOSAMINE DERIVATIVE, AND PREPARATION METHOD THEREFOR AND USE THEREOF**

(30) Priority: 04.06.2021 CN 202110624071
(71) Applicant: Risen (Suzhou) Pharma Tech Co., Ltd., Suzhou, Jiangsu 215000 (CN)
(72) Inventor: LYU, Jiasheng, Suzhou, Jiangsu 215000 (CN); GU, Jiamin, Suzhou, Jiangsu 215000 (CN); GE, Jian, Suzhou, Jiangsu 215000 (CN)
(74) Representative: Regimbeau
(86) International application number: PCT/CN2022/096315
(87) International publication number: WO 2022/253234

(57) **Abstract**

The present invention relates to crystalline forms I and II of the compound 2-N-4,6-di-O-tributyryl-D-glucosamine, preparation methods and uses thereof, wherein the crystalline forms I and II obtained from the present invention have high water solubility, crystalline stability and can be better applied in pharmacy.

## Description

### CROSS-REFERENCE TO RELATED APPLICATION

This application is submitted based on Chinese Patent Application No. 202110624071.9, filed on June 4, 2021, and claims priority to that Chinese patent application, the entire content of which is hereby incorporated by reference.

### FIELD OF THE INVENTION

The present invention relates to crystalline forms I and II of 2-N-4,6-di-O-tributyryl-D-glucosamine, preparation methods and uses thereof.

### BACKGROUND OF THE INVENTION

The structure of crystalline form as an active pharmaceutical ingredient often affects the chemical stability of the medication. Differences in crystallization and storage conditions may lead to changes in the structure of crystalline form of the compound, sometimes accompanied by the production of other crystalline forms. In general, an amorphous pharmaceutical product does not have a regular structure of crystalline form and tends to have other defects, such as poor stability, fine precipitated crystals, difficulty in filtration, easy agglomeration, poor flowability, and the like.

Therefore, the selection of a new pharmaceutically acceptable crystalline form is a critical step in the process of developing a new medication. This is due to the fact that certain crystalline polymorphs of the medications are often important determinants of ease of preparation, solubility, stability during distribution and storage, ease of formulation and pharmacokinetic properties of the active pharmaceutical ingredients (API). Crystalline polymorphs are produced when crystallized in different lattice arrangements with specific thermodynamic properties and stability.

The present inventors have discovered that 2-N-4,6-di-O-tributyryl-D-glucosamine (the structure of which is as follows, Formula I) as shown below is a pharmaceutically active ingredient for preventing or treating osteoarthritis (which is recited in the present applicant's Chinese Patent Application No. CN201711364533.8, and incorporated herein by reference in its entirety).

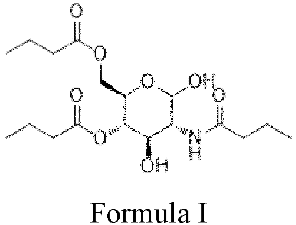

Therefore, it is essential that the crystalline forms of the compound 2-N-4,6-di-O-tributyryl-D-glucosamine and the preparation methods thereof are investigated deeply to improve various aspects of the properties of this glucosamine compound.

### SUMMARY OF THE INVENTION

It is an object of the present invention to provide new crystalline forms of the compound 2-N-4,6-di-O-tributyryl-D-glucosamine, as well as the preparation methods and uses thereof. Among them, compared with the amorphous state of this glucosamine compound, the crystalline forms prepared by the present invention have good water solubility, crystalline stability, low hygroscopicity, etc., which are in line with the quality requirements of pharmaceutical preparations, can be stored for a long period of time, and can be applied in the production of preparations.

In a first aspect, the present invention provides a crystalline form I of the compound shown in formula I below. The crystalline form I has characteristic peaks at diffraction angles of 5.13°, 5.73°, 6.79°, 19.41°, 19.73°, 21.78°, and 23.28° in the X-ray powder diffraction pattern expressed in terms of the angle of diffraction angle 20 obtained using Cu-Kα radiation, wherein each of the diffraction angles 20 has an error range of ±0.2°:

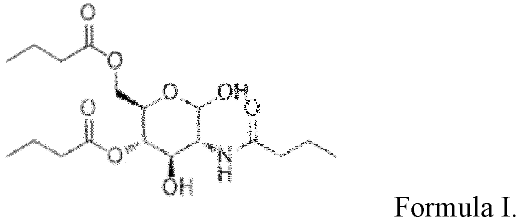

Further, the characteristic peaks are at diffraction angle 20 of 5.13°, 5.73°, 6.79°, 10.28°, 10.85°, 11.49°, 13.58°, 14.49°, 16.23°, 18.14°, 19.41°, 19.73°, 20.97°, 21.78°, 23.28° and 25.07°, wherein each diffraction angle has an error range of ±0.2°. Still further, the characteristic peaks are at diffraction angle 20 of 5.13°, 5.73°, 6.79°, 8.10°, 10.28°, 10.85°, 11.49°, 13.58°, 14.49°, 16.23°, 18.14°, 18.46°, 19.41°, 19.73°, 20.44°, 20.97°, 21.78°, 22.10°, 23.07°, 23.28°, 23.62°, 24.07°, 25.07°, 26.87°, 27.41°, 28.94°, and 39.95°, wherein the 20 for each characteristic peak has an error range of ±0.2°.

Further, in a differential scanning calorimetry, the crystalline form I has an onset temperature of the melting point peak of 109.06°C and exhibits a maximum endothermic peak at 113.563°C.

In a second aspect, the present invention provides a crystalline form II of the compound shown in formula I above, wherein the crystalline form has characteristic peaks at diffraction angles of 3.60°, 4.56°, 5.48°, 6.16°, 9.04°, 10.96°, 20.38°, and 32.97° in the X-ray powder diffraction pattern expressed in terms of the angle of diffraction angle 20 obtained using Cu-Kα radiation, wherein each of the diffraction angle 20 has an error range of ±0.2°.

Further, in a differential scanning calorimetry, the crystalline form II has an onset temperature of the melting point peak of 82.72°C and exhibits a maximum endothermic peak at 92.32°C.

In a third aspect, the present invention also provides a method for preparing crystalline form I. Specifically, the method comprises the steps of:
dissolving the compound shown in formula I in a solvent;
subsequently subjecting the formulated solution to a heating-cooling cycle;
wherein the heating-cooling cycle is performed by;
   equilibrating at 25°C for 5 minutes;
   heating to 80°C at a rate of 1°C/min and holding at 80°C for 30 minutes;
   and then cooling to -20°C at a rate of 1°C/min and holding for 2 hours;
wherein the solvent is acetonitrile.

Alternatively, crystalline form I can be prepared by the following method comprising the steps of:
dissolving the compound shown in formula I in a good solvent to obtain a nearly saturated solution; and lowering the temperature of the solution as completely dissolved to 0°C, then heating the solution from 20°C to 25°C, adding a crystal seed, stirring, and then obtaining a solid crystal; or,
raising the temperature of the solution as completely dissolved to 30°C, then cooling the solution to 25°C, adding a crystal seed, stirring and then obtaining a solid crystal.

In a fourth aspect, the present invention also provides a method for the preparation of crystalline form II. Specifically, the method comprises the following steps of:
dissolving the compound shown in formula I in a good solvent to obtain a nearly saturated solution; and
adding a poor solvent to the nearly saturated solution at 2 to 4 folds the volume of the solution to obtain a suspension, and then stirring the suspension at room temperature overnight;
wherein the good solvent is methyl tert-butyl ether; and the poor solvent is heptane.

In a fifth aspect, the present invention provides a pharmaceutical composition comprising the crystalline form I or II as described above, and a pharmaceutically acceptable carrier.

In a sixth aspect, the present invention provides a use of the crystalline form I or II or pharmaceutical composition as described above in the preparation of a medication for the prevention or treatment of a bone or joint disease. Preferably, the bone or joint disease is osteoporosis, osteopenia and/or arthritis. Among them, the arthritis is, for example, osteoarthritis, inflammatory arthritis (including rheumatoid arthritis or psoriatic arthritis), traumatic arthritis, degenerative arthritis, or dysplastic arthritis.

The crystalline form I or II of the compounds shown in formula I as prepared by the present invention has good stability, high water solubility and purity. Therefore, the medicinal requirements for production, transportation and storage can be met, and the production process is stable, reproducible and controllable, , and is suitable for industrialized production.

### BRIEF DESCRIPTION OF THE DRAWINGS

FIG. 1 illustrates an XRPD pattern of crystalline form I according to the present invention;
FIG. 2 illustrates an XRPD pattern of crystalline form II according to the present invention;
FIG. 3 illustrates an XRPD overlay of crystalline forms I and II according to the present invention and API crystalline form;
FIG. 4 illustrates a DSC overlay of crystalline forms I and II according to the present invention and API crystalline form;
FIG. 5 illustrates a TGA overlay of crystalline forms I and II according to the present invention and API crystalline form;
FIG. 6 illustrates a DVS diagram of crystalline form I according to the present invention;
FIG. 7 illustrates an IR diagram of crystalline form I according to the present invention;
FIG. 8 illustrates a DVS diagram of crystalline form II according to the present invention;
FIG. 9 illustrates a crystal habit diagram of the API crystalline form obtained by scanning electron microscopy;
FIG. 10 illustrates a crystal habit diagram of crystalline form I according to the present invention obtained by scanning electron microscopy;
FIG. 11 illustrates a crystal habit diagram of crystalline form II according to the present invention obtained by scanning electron microscopy;
FIG. 12 illustrates an XRPD overlay obtained from solubility tests in water for crystalline forms I and II according to the present invention and API crystalline form;
FIG. 13 illustrates a solid XRPD overlay obtained from the stability test of crystalline form I according to the present invention;
FIG. 14 illustrates an XRPD overlay obtained from a scale-up experiment of crystalline form I according to the present invention.

### DETAILED DESCRIPTION OF THE INVENTION

In order to provide a clear and consistent understanding of the terms used in the present specification, a number of definitions are provided below. Moreover, unless defined otherwise, all technical and scientific terms as used herein have the same meaning as commonly understood to one of ordinary skill in the art to which this invention pertains.

As used herein, the term "crystalline form" or "crystal" refers to any solid material exhibiting a three-dimensional ordering, which produces a characteristic XRPD pattern with well-defined peaks, as opposed to an amorphous solid material.

As used herein, the term "X-ray powder diffraction pattern (XRPD pattern)" refers the one according to the Bragg's formula 2d sinθ = nλ (where λ is the wavelength of the X-rays, λ = 1.54056 Å; and the diffraction order, n, is any positive integer, generally a first-order diffraction peak, n = 1). When X-rays are incident at a grazing angle θ (the complementary angle of the angle of incidence, also known as the Bragg angle) on certain atomic surface of a crystal or partially crystalline sample with a d-dot matrix plane spacing, the Bragg's equation is satisfied, and then this set of X-ray powder diffraction pattern can be measured. XRPD pattern is usually characterized by peak positions (horizontal coordinates) and/or peak intensities (vertical coordinates).

As used herein, the term "differential scanning calorimetry or DSC" refers to the measurement of the difference in temperature and heat flow between a sample and a reference material during heating or holding the sample, in order to characterize all the physical and chemical changes related to thermal effects and to obtain information about the phase transition of the sample.

As used herein, the term "thermogravimetric analysis or TGA" refers to the measurement of the mass of a sample to be tested as a function of temperature at a programmed temperature in order to study the thermal stability and composition of a material.

As used herein, the term "dynamic vapor sorption or DVS" refers to an accurate characterization of the moisture sorption and interaction properties of a material using gravimetric principles.

As used herein, the term "20 or 20 angle" refers to a peak position expressed in degrees based on the setup in an X-ray diffraction experiment and is usually the unit of the horizontal coordinate in the diffraction pattern. If the reflection is diffracted when the incident beam forms a θ angle with certain lattice plane, the reflected beam is required to be recorded with a 20 angle in the experimental setup. If not specified, the 20 value has an error range of ±0.2°.

As used herein, the term "crystal habit" refers to the macroscopic external morphology of crystals, which may be different for the same crystal obtained by different crystallization methods. It is usually measured by scanning electron microscopy.

The present application will be further described hereinafter in connection with examples, which are used only to illustrate the technical solutions of the present invention and are not intended to limit the essence and scope of the present invention. In addition, the reagents used in the following examples are commercially available.

### Comparative Example 1: Preparation method of Example 16 in CN201711364533.8

Step 1: 4,6-*O*-benzylidene-2-*N*-butyryl-D-glucosamine (3.37 g, 10 mmol, 1 eq) was dispersed into 50 mL of DMF and cooled to -10°C under nitrogen protection. NaH (1.08 g, 27 mmol, 2.7 eq) was added portion wise upon stirring while keeping the temperature of the reaction system below 0°C. The reaction system was slowly brought to room temperature and stirred for 2 hours at room temperature. The reaction solution was poured into 300 mL of water and the mixture was stirred for 1 hour. The filter cake was obtained via suction filtration, then washed with 50 mL of water, followed by washing with 50 mL of petroleum ether, and dried to obtain 4,6-*O*-benzylidene-1,3-di-*O*-dibenzyl-2-*N*-butyryl-D-glucosamine (4.5 g, 87.1% yield).

Step 2: D-glucosamine (4.5 g, 8.7 mmol, 1 eq) obtained in Step 1 was dispersed into 90 mL of dichloromethane, 1.5 mL of water and 18 mL of trifluoroacetic acid were added upon stirring, and the mixture was stirred for 10 minutes at room temperature. 50 mL of water was added and stirred, and then the organic phase was separated. The organic phase was washed with 50 mL of water, followed by washing with 50 mL of saturated sodium bicarbonate aqueous solution. The solvent was dried via rotary evaporation and the residue was slurried with hot petroleum ether, filtered and dried to obtain 1,3-di-*O*-dibenzyl-2-*N*-butyryl-D-glucosamine (3.2 g, 85.7% yield).

Step 3: D-glucosamine (2.5 g, 5.8 mmol, 1 eq) obtained in Step 2 was dispersed into 25 mL of pyridine and DMAP (0.04 g, 0.29 mmol, 0.05 eq) and butyric anhydride (2.3 g, 14.5 mmol, 2.5 eq) were added upon stirring. After stirring for 16 hours at room temperature, the reaction solution was poured into 250 mL of water and the mixture was stirred for 1 hour. The filter cake was obtained via suction filtration, then washed with 50 mL of water, followed by washing with 50 mL of petroleum ether, and dried to obtain 1,3-di-*O*-dibenzyl-2-*N*-4,6-di-*O*-tributyryl-D-glucosamine (2.9 g, 87.8% yield).

Step 4: D-glucosamine (2.9 g) obtained in Step 3 was dispersed into 15 mL of methanol, 1.45 g of 10% palladium carbon was added, and 15 mL of acetic acid was added. After hydrogen displacement, the mixture was stirred in a hydrogen atmosphere for 48 hours. The reaction mixture was filtered, the filtrate was dried via rotary evaporation, and the residue was purified by column chromatography (MeOH/DCM=1/100-1/30) to obtain 2-N-4,6-di-O-tributyryl-D-glucosamine (1.3 g, 65.6% yield).

MS (m/z (ESI⁺)) 389.8.

The solid product was detected by scanning electron microscopy as an API crystalline form and the crystal habit diagram thereof is shown in FIG. 9.

### Example 1. Preparation of Crystalline Form I

About 100 mg of the product 2-N-4,6-di-O-tributyryl-D-glucosamine obtained from Comparative Example 1 was dissolved in a 4 ml vial containing 300 µL of acetonitrile. The solution was then subjected to a heating-cooling cycle comprising the following procedures of:
equilibrating at 25°C for 5 minutes
heating to 80°C at a rate of 1°C/min and holding for 30 minutes;
and then cooling to -20 °C at a rate of 1°C/min and holding for 2 hours.

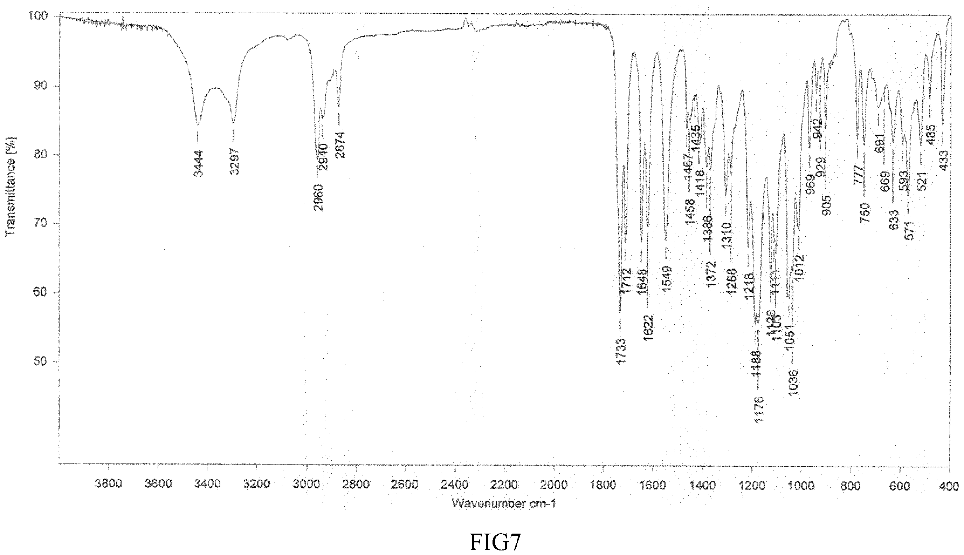

When the above temperature program was completed, the vial was brought to room temperature and stirred overnight to obtain solid I (crystalline form I). The resulting solid was subjected to infrared testing after infrared tableting. The results as illustrated are shown in FIG. 7.

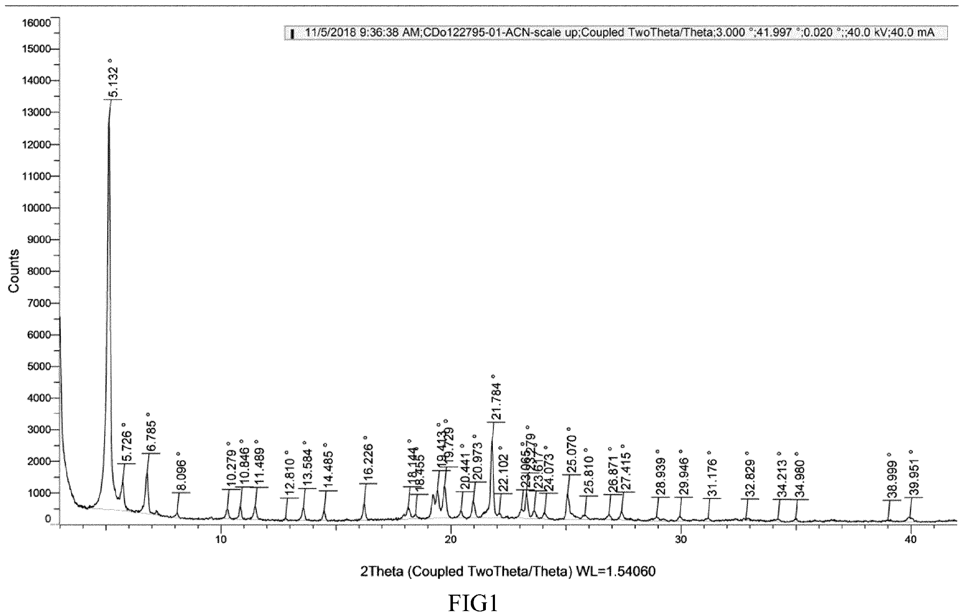

In addition, an XRPD pattern as shown in FIG. 1 (test conditions are shown in detail below) was obtained via a Bruker D8 Focus instrument for this solid sample, with the characteristic peak information shown in Table 1 below.

**Table 1. Characteristic peaks of crystalline form I**

| **No.** | **2θ [°]** | **d [Å]** | **Net intensity** | **Total intensity** | **Relative intensity** | **FWHM** | **Width (low)** |
|---|---|---|---|---|---|---|---|
| 1 | 5.132 ° | 17.20596 Å | 12292 | 12770 | 100.0 % | 0.143 | 0.0717 |
| 2 | 5.726 ° | 15.42213 Å | 860 | 1298 | 7.0 % | 0.182 | 0.0910 |
| 3 | 6.785 ° | 13.01755 Å | 1276 | 1623 | 10.4 % | 0.126 | 0.0631 |
| 4 | 8.096 ° | 10.91252 Å | 164 | 382 | 1.3 % | 0.115 | 0.0577 |
| 5 | 10.279 ° | 8.59934 Å | 312 | 487 | 2.5 % | 0.132 | 0.0659 |
| 6 | 10.846 ° | 8.15044 Å | 391 | 561 | 3.2 % | 0.107 | 0.0533 |
| 7 | 11.489 ° | 7.69567 Å | 392 | 551 | 3.2 % | 0.129 | 0.0643 |
| 8 | 12.810 ° | 6.90528 Å | 78.6 | 209 | 0.6 % | 0.078 | 0.0391 |
| 9 | 13.584 ° | 6.51325 Å | 381 | 515 | 3.1 % | 0.129 | 0.0645 |
| 10 | 14.485 ° | 6.11028 Å | 310 | 441 | 2.5 % | 0.101 | 0.0504 |
| 11 | 16.226 ° | 5.45815 Å | 526 | 671 | 4.3 % | 0.111 | 0.0554 |
| 12 | 18.144 ° | 4.88525 Å | 392 | 564 | 3.2 % | 0.157 | 0.0784 |
| 13 | 18.455 ° | 4.80366 Å | 145 | 325 | 1.2 % | 0.507 | 0.2534 |
| 14 | 19.413 ° | 4.56868 Å | 880 | 1079 | 7.2 % | 0.412 | 0.2058 |
| 15 | 19.729 ° | 4.49623 Å | 992 | 1196 | 8.1 % | 0.162 | 0.0810 |
| 16 | 20.441 ° | 4.34130 Å | 208 | 416 | 1.7 % | 0.147 | 0.0737 |
| 17 | 20.973 ° | 4.23224 Å | 509 | 720 | 4.1 % | 0.131 | 0.0654 |
| 18 | 21.784 ° | 4.07662 Å | 2396 | 2611 | 19.5 % | 0.127 | 0.0633 |
| 19 | 22.102 ° | 4.01859 Å | 140 | 352 | 1.1 % | 0.160 | 0.0800 |
| 20 | 23.065 ° | 3.85302 Å | 276 | 473 | 2.2 % | 0.162 | 0.0811 |
| 21 | 23.279 ° | 3.81800 Å | 811 | 1005 | 6.6 % | 0.127 | 0.0636 |
| 22 | 23.617 ° | 3.76423 Å | 259 | 445 | 2.1 % | 0.155 | 0.0775 |
| 23 | 24.073 ° | 3.69390 Å | 187 | 359 | 1.5 % | 0.278 | 0.1388 |
| 24 | 25.070 ° | 3.54923 Å | 779 | 945 | 6.3 % | 0.145 | 0.0723 |
| 25 | 25.810 ° | 3.44905 Å | 109 | 280 | 0.9 % | 0.412 | 0.2059 |
| 26 | 26.871 ° | 3.31522 Å | 160 | 319 | 1.3 % | 0.181 | 0.0907 |
| 27 | 27.415 ° | 3.25069 Å | 256 | 405 | 2.1 % | 0.135 | 0.0676 |
| 28 | 28.939 ° | 3.08286 Å | 117 | 239 | 1.0 % | 0.346 | 0.1731 |
| 29 | 29.946 ° | 2.98148 Å | 114 | 238 | 0.9 % | 0.157 | 0.0786 |
| 30 | 31.176 ° | 2.86653 Å | 79.1 | 201 | 0.6 % | 0.174 | 0.0868 |
| 31 | 32.829 ° | 2.72592 Å | 53.5 | 183 | 0.4 % | 0.251 | 0.1254 |
| 32 | 34.213 ° | 2.61873 Å | 69.3 | 171 | 0.6 % | 0.248 | 0.1241 |
| 33 | 34.980 ° | 2.56303 Å | 67.2 | 161 | 0.5 % | 0.123 | 0.0615 |
| 34 | 38.999 ° | 2.30770 Å | 37.3 | 142 | 0.3 % | 0.141 | 0.0704 |
| 35 | 39.951 ° | 2.25487 Å | 128 | 224 | 1.0 % | 0.278 | 0.1389 |

### Example 2. Preparation of Crystalline Form II

About 60 mg of the product 2-N-4,6-di-O-tributyryl-D-glucosamine obtained from Comparative Example 1 was dissolved in 2 ml of methyl tert-butyl ether to obtain a nearly saturated and clear solution, and 5 ml of heptane was added to the solution to induce precipitation.

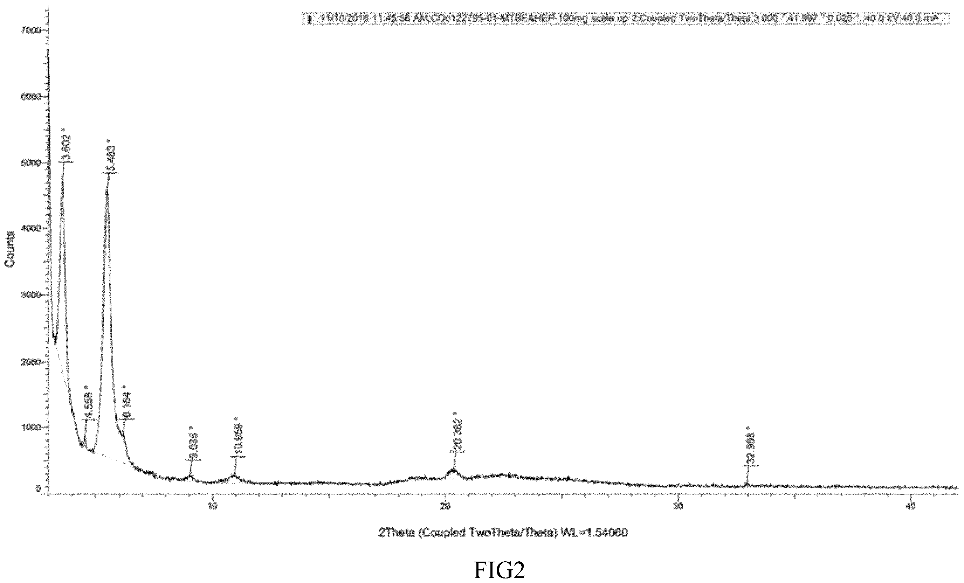

The suspension was slurried overnight at room temperature and then filtered to obtain a solid (crystalline form II). An XRPD pattern as shown in FIG. 2 (test conditions are shown in detail below) was obtained via a Bruker D8 Focus instrument for this solid sample, with the characteristic peak information shown in Table 2 below.

**Table 2. Characteristic peaks of crystalline form II**

| **No.** | **2θ [°]** | **d [Å]** | **Net intensity** | **Total intensity** | **Relative intensity** | **FWHM** | **Width (low)** |
|---|---|---|---|---|---|---|---|
| 1 | 3.602 ° | 24.51240 Å | 2912 | 4766 | 72.2 % | 0.238 | 0.1190 |
| 2 | 4.558 ° | 19.37172 Å | 160 | 867 | 4.0 % | 0.076 | 0.0379 |
| 3 | 5.483 ° | 16.10612 Å | 4035 | 4599 | 100.0 % | 0.312 | 0.1562 |
| 4 | 6.164 ° | 14.32737 Å | 410 | 874 | 10.2 % | 0.160 | 0.0800 |
| 5 | 9.035 ° | 9.77972 Å | 66.5 | 255 | 1.6 % | 0.270 | 0.1348 |
| 6 | 10.959 ° | | 146 | 310 | 3.6 % | 0.294 | 0.1472 |
| 7 | 20.382 ° | 4.35372 Å | 162 | 387 | 4.0 % | 0.431 | 0.2156 |
| 8 | 32.968 ° | 2.71477 Å | 65.0 | 173 | 1.6 % | 0.114 | 0.0572 |

### Example 3. Evaluation of the Physicochemical Properties of Three Crystalline Forms

The API crystalline form (amorphous) as well as the crystalline forms I and II of the present invention were analyzed by X-ray powder diffraction (XRPD), differential scanning calorimetry (DSC), thermogravimetric analysis (TGA), dynamic vapor sorption (DVS) and scanning electron microscopy (SEM), respectively. During each of these analyses, it should be understood that the results obtained can vary within acceptable instrumental and operational errors. In differential scanning calorimetry (DSC), for example, the maximum endothermic peak obtained may vary within the range of less than ±5°C, such as ±3°C, ±2°C or ±1°C.

The specific conditions or parameters of the above analyses are shown as follows.

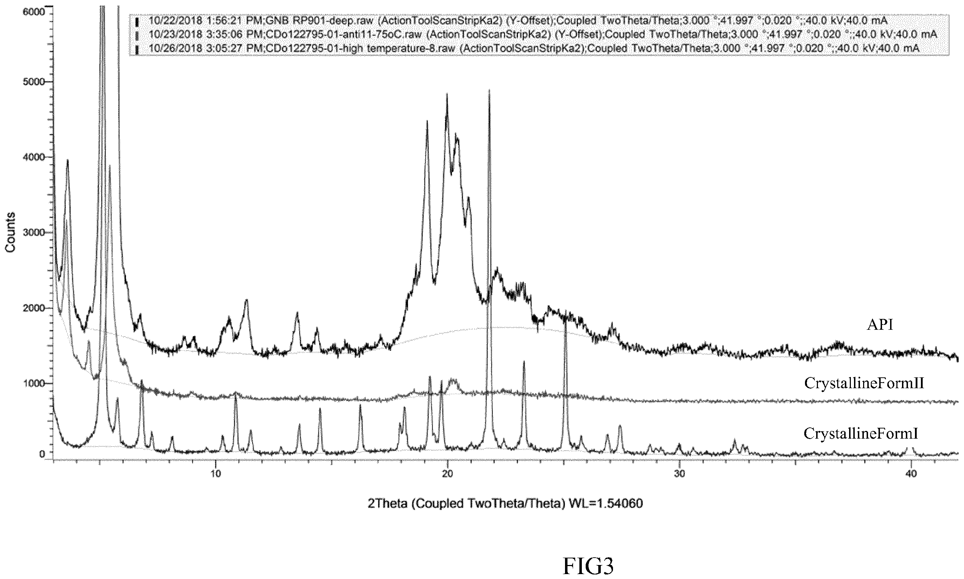

XRPD: The samples were analyzed using a Bruker D8 Focus instrument, in which the 20 scanning angles ranged from 3° to 42°, with a scanning step size of 0.02°, and a scanning time of 0.2 s for each step. The phototube voltage and current were 40 kV and 40 mA, respectively. In the preparation, an appropriate amount of sample was placed in a sample tray, and flattened with a tool such as a spoon or a glass piece to ensure that its surface is smooth and flat. The resulting X-ray powder diffraction (XRPD) patterns are shown in FIGs. 1 to 3.

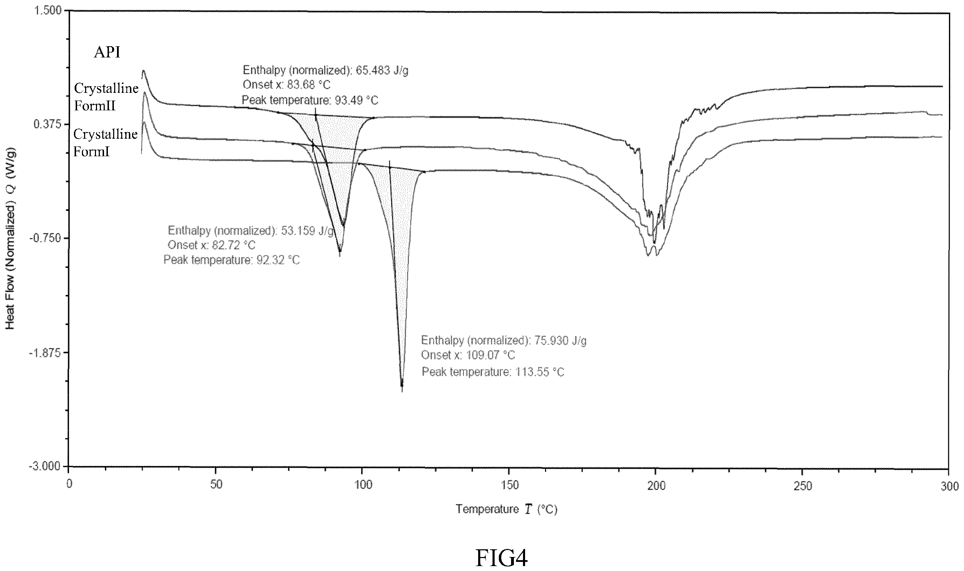

DSC: Samples were analyzed using a TA Instruments Discovery DSC 25. The weighed samples were placed in a sample tray and brought to the specified temperature at a rate of 10°C/min under nitrogen protection (50 ml/min). In the present invention, the DSC graphs of the above three samples are illustrated in the form of overlay, as shown in FIG. 4.

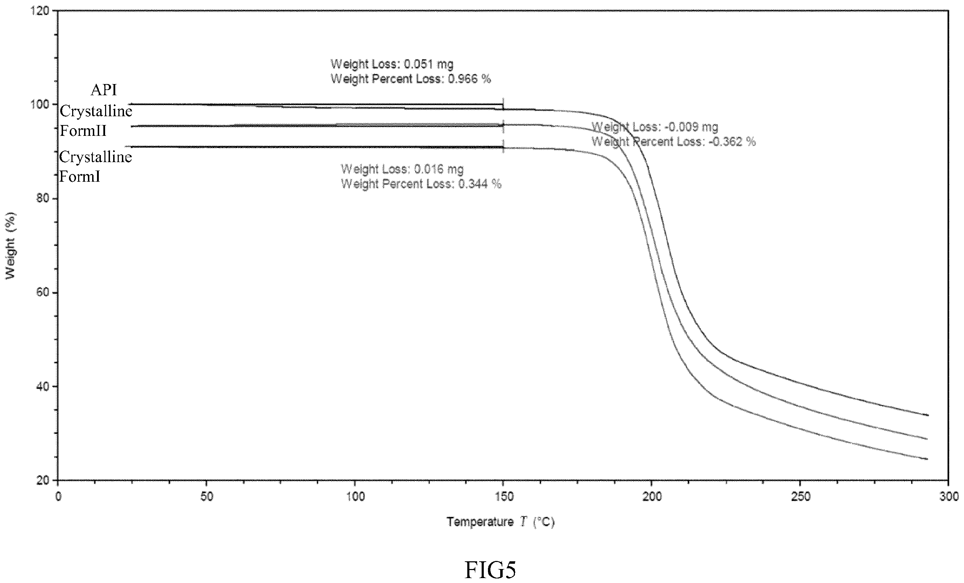

TGA: Samples were analyzed using a TA Instruments TGA Discovery 550. The sample were placed in a tared aluminum tray and automatically weighed, and then the samples were brought to the specified temperatures at a rate of 10°C/min under the protection of nitrogen. In the present invention, the TGA graphs of the above three samples are illustrated in the form of overlay, as shown in FIG. 5.

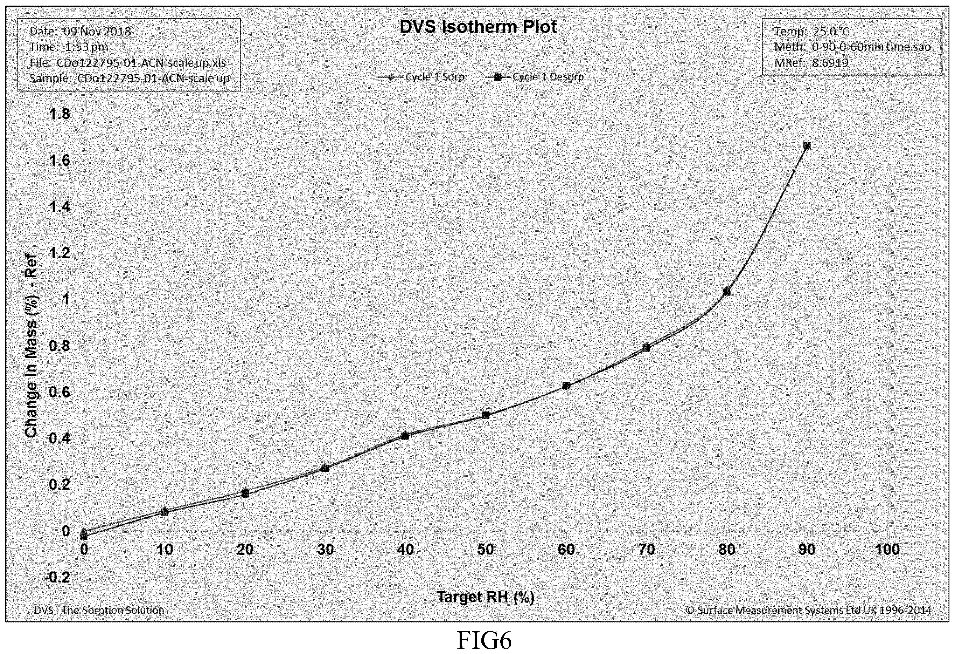

DVS: Samples were analyzed using an Intrinsic DVS (System Measurement System UK). The amount of test sample was about 20-30 mg. The temperature of the test chamber was controlled between 25±1°C, the relative humidity was increased from 0% to 90% and then decreased to 0% at a rate of 10%/h, and the quality data was recorded every 20s. DVS diagrams of the resulting crystalline forms I and II are shown in FIGs. 6 and 8, respectively. The relevant data are presented in Tables 4 and 5.

SEM: Samples were analyzed using a Phenom pure+. The sample was sprayed with gold and then placed in the instrument for testing. Different magnifications were adjusted to obtain the crystal habits of the samples and the results are shown in FIGs. 9 to 11.

The results as illustrated are shown in Table 3 below and FIGs. 4 to 9.

**Table 3: Summary of physicochemical properties of three crystalline forms of the compound of formula I**

| | API | Crystalline Form II | Crystalline Form I |
|---|---|---|---|
| TGA weight loss (wt%) | 0.966 | None | 0.344 |
| Melting point/°C | 93.49 | 92.32 | 113.55 |
| DVS weight gain at an RH of 80% | 2.440% | 0.869% | 1.037% |

**Table 4. DSV data of crystalline form I**

| | **Objective RH (%)** | **Sorption** | **Mass change (%) - ref Desorption** | **Delay** |
|---|---|---|---|---|
| Cycle 1 | 0.0 | 0.001 | -0.022 | |
| | 10.0 | 0.092 | 0.082 | -0.010 |
| | 20.0 | 0.175 | 0.160 | -0.015 |
| | 30.0 | 0.276 | 0.270 | -0.006 |
| | 40.0 | 0.417 | 0.409 | -0.007 |
| | 50.0 | 0.502 | 0.498 | -0.003 |
| | 60.0 | 0.626 | 0.627 | 0.001 |
| | 70.0 | 0.800 | 0.789 | -0.011 |
| | 80.0 | 1.037 | 1.031 | -0.006 |
| | 90.0 | 1.662 | 1.662 | |

**Table 5. DSV data of crystalline form II**

| | **Objective RH (%)** | **Sorption** | **Mass change (%) - ref Desorption** | **Delay** |
|---|---|---|---|---|
| Cycle 1 | 0.0 | 0.000 | 0.023 | |
| | 10.0 | 0.086 | 0.552 | 0.466 |
| | 20.0 | 0.165 | 0.659 | 0.494 |
| | 30.0 | 0.257 | 0.777 | 0.520 |
| | 40.0 | 0.324 | 0.913 | 0.590 |
| | 50.0 | 0.368 | 0.968 | 0.600 |
| | 60.0 | 0.457 | 1.080 | 0.623 |
| | 70.0 | 0.629 | 1.214 | 0.586 |
| | 80.0 | 0.869 | 1.387 | 0.518 |
| | 90.0 | 1.820 | 1.820 | |

### Example 4. Solubility Test

The excess crystals were dissolved in an amount of purified water in a 4 ml vial at room temperature. The mass of an empty watch glass was recorded as m1. The suspension was stirred for 2 hours and then filtered through a 0.22 µL membrane into an empty watch glass, the mass m2 at this point was recorded for this watch glass, and the remaining solid was used for XRPD analysis.

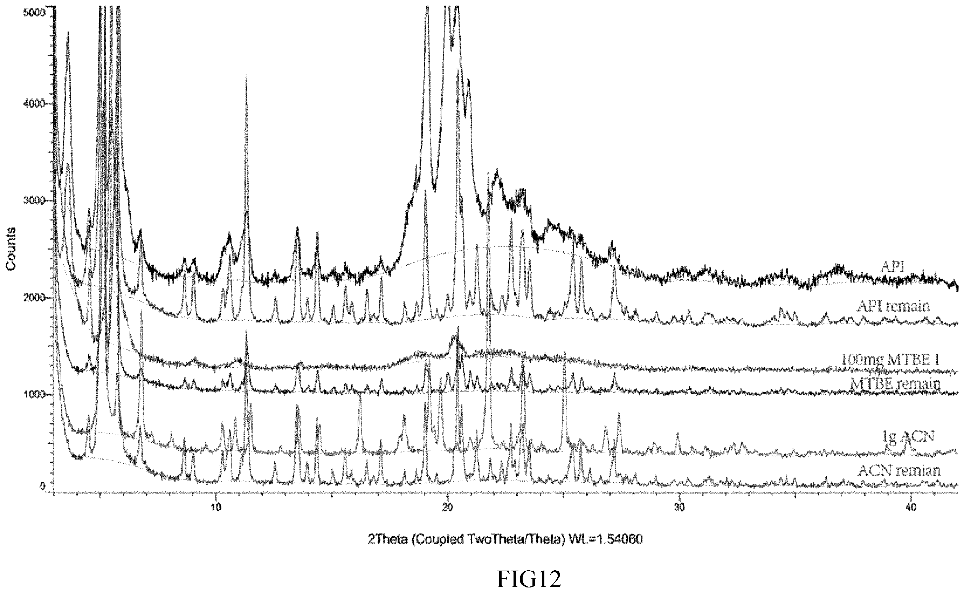

The watch glass containing the filtrate was dried in an oven at 40°C. After drying, the mass m3 was recorded and the solubility data of the substrate could be calculated. The masses of the watch glass as well as the amounts of solid and solution are listed in Table 6, and the experimental results are shown in Table 7 and FIG. 12.

**Table 6. Experimental parameters of solubility in water for the three crystalline forms**

| | API | Crystalline Form II | Crystalline Form I |
|---|---|---|---|
| Sample/mg | 53.10 | 27.05 | 51.22 |
| Solvent/ml | 3 | 2 | 3.5 |
| m1/g | 13.5946 | 10.8655 | 13.8064 |
| m2/g | 15.5383 | 12.5220 | 16.3467 |
| m3/g | 13.6114 | 10.8804 | 13.8291 |

**Table 7. Solubility in water for the three crystalline forms**

| | |
|---|---|
| API/mg/g | 8.12 |
| Crystalline Form II/mg/g | 9.08 |
| Crystalline Form I/mg/g | 9.02 |

### Example 5. Testing of the Solubility in Buffer of Crystalline Form I of the Present Invention

The solubility of crystalline form I of the present invention was investigated at room temperature in buffers at pH 1.2, 4.5 and 6.8. The procedure was consistent with that of the solubility test in water. The masses of the watch glass as well as the amounts of solid and solution are listed in Table 8, and the experimental results are shown in Table 9.

**Table 8. Experimental parameters of solubility in buffer for crystalline form I**

| | | | |
|---|---|---|---|
| pH | 1.2 | 4.5 | 6.8 |
| Sample/mg | 28.4 | 31.9 | 30.2 |
| Solvent/ml | 3 | 3 | 3 |
| m1/g | 21216.83 | 30049.19 | 30816.08 |
| m2/g | 24148.45 | 32885.13 | 33642.83 |
| m3/g | 21248.69 | 30080.75 | 30865.30 |

**Table 9. Solubility in buffer for crystalline form I**

| | |
|---|---|
| pH:1.2/mg/g | 4.08 |
| pH:4.5/ mg/g | 6.58 |
| pH:6.8/ mg/g | 10.02 |

### Example 6. Stability Test

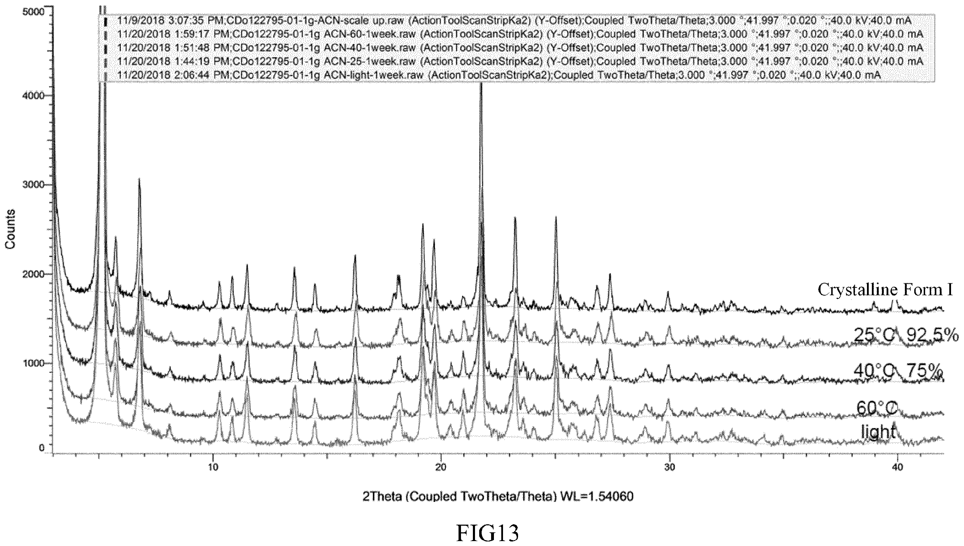

The stability of crystalline form I was measured under the temperature and humidity conditions shown in Table 10. 10 mg of the solid was placed into a 2 ml glass vial and the vial was transferred to a stability chamber. The experimental conditions were 25°C/92.5% RH, 40°C/75% RH, 60°C, and light, respectively. The solid was removed after one week for determining XRPD and the results are shown in Table 10 and FIG. 13.

**Table 10. Stability test results**

| No. | Condition | Whether the crystalline form is changed |
|---|---|---|
| 1 | 25°C,92.5%RH | No |
| 2 | 40°C,75%RH | No |
| 3 | 60°C | No |
| 4 | Light | No |

### Example 7. Scale-up experiments of crystalline form I

Example 7.1: 300 mg of API solid was dissolved in 0.8 ml of acetonitrile at room temperature to obtain a nearly saturated solution. The formulated solution was placed in crystal 16 and heated and cooled with the temperature program as shown in Example 1. When the temperature program was completed, the vial was brought to room temperature and stirred overnight. The resulting solids were subjected to XRPD assay.

Example 7.2: 1000 mg of API solid was dissolved in 1.5 ml of acetonitrile at room temperature to obtain a nearly saturated solution. The solution was cooled to 0°C and became viscous after one hour. The solution was then heated to 20°C and recovered to be clear, 4.83 mg of crystalline seed was added to the solution and stirred for 3 hours. The resulting solids were subjected to XRPD assay. Example 7.3: 1200 mg of API solid was dissolved in 2 ml of acetonitrile at room temperature to obtain a nearly saturated solution. The solution was cooled to 0°C and became viscous after one hour. The solution was then heated to 25°C and recovered to be clear, an amount of crystalline seed was added to the solution and stirred for 3 hours. The resulting solids were subjected to XRPD assay. Example 7.4: 3100 mg of API solid was dissolved in 4.9 ml of acetonitrile at room temperature to obtain a nearly saturated solution. The solution was heated to 30°C to ensure that the solid was completely soluble in the solution. The solution was then cooled to 25°C and still clear, an amount of crystalline seed was added to the solution and stirred for 1 hour. The resulting solids were subjected to XRPD assay.

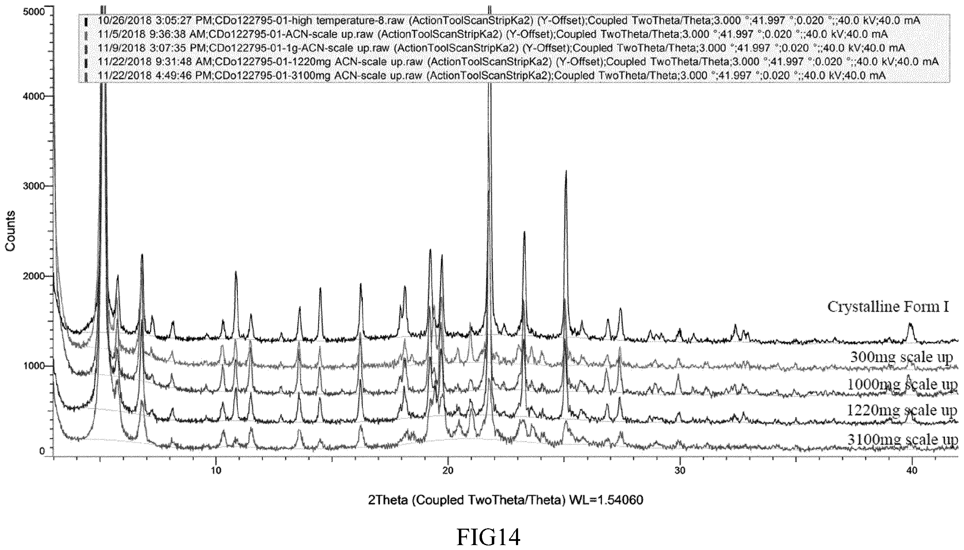

The XRPD patterns of Example 7.1 to Example 7.4 above are shown in FIG. 14. It can be seen that both new crystalline forms have almost no weight loss until 150°C. The solubility of new crystalline forms I and II in water was 9.08 mg/g and 9.02 mg/g, respectively. As can be seen from the above data, these two new crystalline forms have excellent stability, which is more in line with the quality requirements for pharmaceutical manufacturing and facilitates long-term preservation.

The present invention is not limited by the examples shown and described above, and is changeable within the scope of the claims.

## Claims

1. A crystalline form I of a compound shown in the following formula I, wherein the crystalline form has characteristic peaks at diffraction angles of 5.13°, 5.73°, 6.79°, 19.41°, 19.73°, 21.78°, and 23.28° in the X-ray powder diffraction pattern expressed in terms of the angle of diffraction angle 20 obtained using Cu-Kα radiation, wherein each diffraction angle has an error range of ±0.2°:

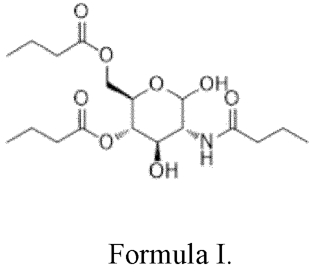

2. The crystalline form I according to claim 1, wherein the characteristic peaks are at diffraction angle 20 of 5.13°, 5.73°, 6.79°, 10.28°, 10.85°, 11.49°, 13.58°, 14.49°, 16.23°, 18.14°, 19.41°, 19.73°, 20.97°, 21.78°, 23.28° and 25.07°, wherein each diffraction angle has an error range of ±0.2°.

3. The crystalline form I according to claim 1, wherein the characteristic peaks are at diffraction angle 20 of 5.13°, 5.73°, 6.79°, 8.10°, 10.28°, 10.85°, 11.49°, 13.58°, 14.49°, 16.23°, 18.14°, 18.46°, 19.41°, 19.73°, 20.44°, 20.97°, 21.78°, 22.10°, 23.07°, 23.28°, 23.62°, 24.07°, 25.07°, 26.87°, 27.41°, 28.94°, and 39.95°, wherein each diffraction angle has an error range of ±0.2°.

4. The crystalline form I according to any of claims 1-3, wherein the crystalline form I has an onset temperature of the melting point peak of 109.06°C and exhibits a maximum endothermic peak at 113.563°C in a differential scanning calorimetry.

5. A crystalline form II of a compound shown in the following formula I, wherein the crystalline form II has characteristic peaks at diffraction angles of 3.60°, 4.56°, 5.48°, 6.16°, 9.04°, 10.96°, 20.38°, and 32.97° in the X-ray powder diffraction pattern expressed in terms of the angle of diffraction angle 20 obtained using Cu-Kα radiation, wherein each diffraction angle has an error range of ±0.2°:

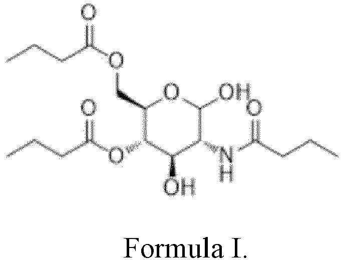

6. The crystalline form II according to claim 5, wherein the crystalline form II has an onset temperature of the melting point peak of 82.72°C and exhibits a maximum endothermic peak at 92.32°C in a differential scanning calorimetry.

7. A method for preparing the crystalline form I according to any of claims 1-4, wherein the method comprises the steps of:
dissolving the compound shown in formula I in a solvent;
subsequently subjecting the formulated solution to a heating-cooling cycle; wherein the heating-cooling cycle is performed by:
equilibrating at 25°C for 5 minutes;
heating to 80°C at a rate of 1°C/min and holding at 80°C for 30 minutes;
and then cooling to -20°C at a rate of 1°C/min and holding for 2 hours;
wherein the solvent is acetonitrile.

8. A method for preparing the crystalline form I according to any of claims 1-4, wherein the method comprises the following steps of:
dissolving the compound shown in formula I in a good solvent to obtain a nearly saturated solution; and
lowering the temperature of the solution as completely dissolved to 0°C, then heating the solution from 20°C to 25°C, adding a crystal seed, stirring and then obtaining a solid crystal; or,
raising the temperature of the solution as completely dissolved to 30°C, then cooling the solution to 25°C, adding a crystal seed, stirring and then obtaining a solid crystal.

9. A method for preparing the crystalline form II according to claim 5 or 6, wherein the method comprises the steps of:
dissolving the compound shown in formula I in a good solvent to obtain a nearly saturated solution; and
adding a poor solvent to the nearly saturated solution at 2 to 4 folds the volume of the solution to obtain a suspension, and then stirring the suspension at room temperature overnight;
wherein the good solvent is methyl tert-butyl ether; and the poor solvent is heptane.

10. A pharmaceutical composition comprising the crystalline form I according to any of claims 1-4 or the crystalline form II according to any of claims 5-6, and a pharmaceutically acceptable carrier.

11. Use of the crystalline form I according to any of claims 1-4 or the crystalline form II according to any of claims 5-6 or the pharmaceutical composition according to claim 10 in the preparation of a medication for the prevention or treatment of a bone or joint disease.

12. The use according to claim 10, wherein the bone or joint disease is osteoporosis, osteopenia and/or arthritis.

13. The use according to claim 11, wherein the arthritis is osteoarthritis, inflammatory arthritis (including rheumatoid arthritis or psoriatic arthritis), traumatic arthritis, degenerative arthritis, or dysplastic arthritis.
